# EUROPEAN PATENT SPECIFICATION

(11) **EP 3 633 592 B1**
(45) Date of publication and mention of the grant of the patent: **09.10.2024**
(21) Application number: 18805204.7
(22) Date of filing: 22.05.2018
(51) Int. Cl.: G06Q 50/10, G06Q 10/00

(54) **INFORMATION PROCESSING DEVICE, PROGRAM, AND INFORMATION PROCESSING SYSTEM**
INFORMATIONSVERARBEITUNGSVORRICHTUNG, PROGRAMM UND INFORMATIONSVERARBEITUNGSSYSTEM
DISPOSITIF DE TRAITEMENT D'INFORMATIONS, PROGRAMME ET SYSTÈME DE TRAITEMENT D'INFORMATIONS

(30) Priority: 23.05.2017 JP 2017101333
(43) Date of publication of application: 08.04.2020
(73) Proprietor: Shiseido Company, Ltd., Chuo-ku Tokyo 104-0061 (JP)
(72) Inventor: SAKAI, Kayoko, Tokyo 104-0061 (JP)
(74) Representative: Müller-Boré & Partner Patentanwälte PartG mbB
(86) International application number: PCT/JP2018/019603
(87) International publication number: WO 2018/216678

(56) References cited:
- WO-A1-2016/203461
- JP-A- 2001 137 216
- JP-A- 2004 121 530
- JP-A- 2006 345 162
- JP-A- 2013 191 203
- JP-U- 3 152 026
- US-A1- 2015 186 518

## Description

### [Technical Field]

The present invention relates to an information processing apparatus, a program, and an information processing system.

### [Background Art]

In general, optimal skin care depends on the specific circumstances of the user. Conventionally, techniques for providing advice on skin care are known.

For example, Patent Literature 1 discloses a technique for displaying advice according to a trouble designated by a user. [Prior Art Documents] WO 2016/203461 A1 relates to a method of dynamically adapting a facial treatment based on a current facial skin profile, comprising: using at least one sensor for measuring at least one current value of at least one variable skin characteristic of facial skin of a patient; acquiring at least one personal skin characteristic of facial skin of the patient; calculating a current facial skin status of the patient according to the at least one personal skin characteristic and the at least one current value; determining instructions to operate a treatment applicator according to the current facial skin status; and instructing the treatment applicator according to the instructions. US 2015/186518 A1 relates to service which can be offered free of charge and the cost of a skin condition measuring device which can be reduced by using data on the occasion of obtaining an analysis result by transmitting measurement data by the skin condition measuring device to a server of a company providing a service of analysing the measurement data. When a request is made from a contractor client to acquire data registered in a measurement data database, authentication is executed based on a contractor ID input from the contractor client.

### [Patent Documents]

[Patent Document 1] Japanese Patent Application Publication No. 2002-056281

### [Summary of Invention]

### [Technical Problem]

The skin is affected by the user's living environment.

That is, it is preferable to consider skin care in consideration of the user's living environment.

However, Patent Document 1 provides advice according to the trouble designated by the user, but does not consider the user's living environment.

Therefore, it is not possible to provide advice according to the user's living environment.

The object of this invention is providing a user with the advice according to a user's living environment.

### [Solution to Problem]

One aspect of the present invention is an information processing apparatus comprising:
a retrieve module configured to retrieve living environment information related to user's living environment;
a determination module configured to determine a skin level based on the retrieved living environment information;
a selection module configured to select advice information associated with the determined skin level among a plurality of pieces of advice information associated with the skin level, each piece of advice information including advice related to the user's skin care; and
a presentation module configured to present the selected advice information.

### [Advantageous Effects of Invention]

According to this invention, the advice according to a user's living environment can be provided to a user.

### [Brief Description of Drawings]

FIG. 1 is a block diagram showing the configuration of an information processing system according to an embodiment of the present invention.
FIG. 2 is a diagram showing a function of an application according to the present embodiment.
FIG. 3 is a diagram showing a data structure of a user information database according to the present embodiment.
FIG. 4 is a diagram showing a data structure of a living environment information database according to the present embodiment.
FIG. 5 is a diagram showing a data structure of an advice information database according to the present embodiment.
FIG. 6 is a flowchart of information processing according to the embodiment.
FIG. 7 is a diagram showing an example of a screen displayed in the information processing of the present embodiment.

### [Description of Embodiments]

Hereinafter, an embodiment of the present invention is described in detail based on the drawings.

Note that, in the drawings for describing the embodiments, the same components are denoted by the same reference sign in principle, and the repetitive description thereof is omitted.

### (1) Configuration of information processing system

The configuration of the information processing system is described.

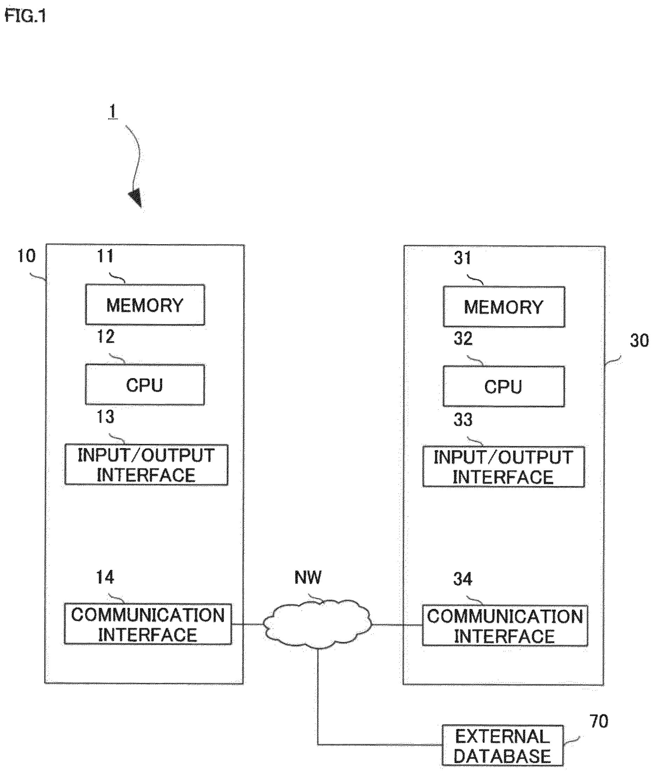

FIG. 1 is a block diagram showing the configuration of the information processing system according to the present embodiment.

As illustrated in FIG. 1, the information processing system 1 includes a client terminal 10, a server 30, and an external database 70.

The client terminal 10 and the server 30 are connected via a network (for example, an Internet or an intranet) NW.

The client terminal 10 is an example of an information processing apparatus that transmits request data to the server 30.

The client terminal 10 is, for example, a smartphone, a tablet device, or a personal computer.

The server 30 is an example of an information processing apparatus that provides response data corresponding to the request data transmitted from the client terminal 10 to the client terminal 10.

The server 30 is, for example, a web server.

The external database 70 is a database that can be accessed by the server 30.

The external database 70 stores information (hereinafter referred to as "living environment information") about the user's living environment for each location.

The living environment information is, for example, information indicating air temperature, humidity, an index (hereinafter referred to as "ultraviolet index") of ultraviolet intensity, and information indicating the amount of scattered pollen.

### (1-1) Configuration of client terminal

The configuration of the client terminal 10 is described with reference to FIG. 1.

As illustrated in FIG. 1, the client terminal 10 includes a memory 11, a CPU (Central Processing Unit) 12, an input/output interface 13, and a communication interface 14.

The memory 11 is configured to store a program and data.

The memory 11 is, for example, a combination of a ROM (read only memory), a RAM (random access memory), and a storage (for example, a flash memory or a hard disk).

The program is, for example, an OS (Operating System) program or an application program that executes information processing.

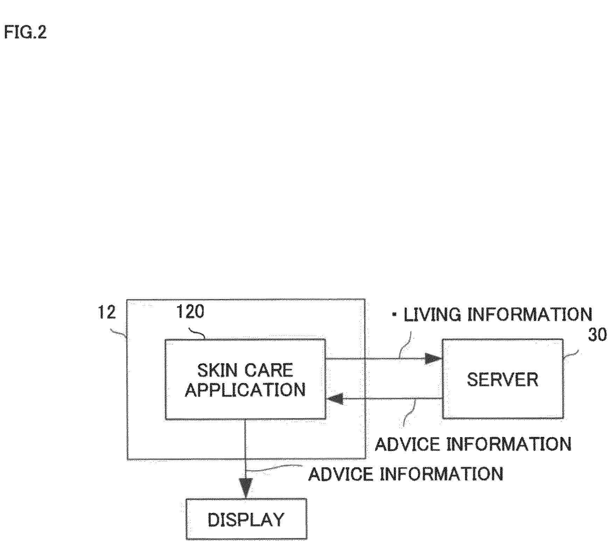

The application includes a skin care application 120 (FIG. 2).

The data includes, for example, the following data:
- Data referenced in an information processing; and
- Data obtained by executing an information processing (that is, an execution result of an information processing).

The CPU 12 is configured to realize the function of the client terminal 10 by starting a program stored in the memory 11.

The input/output interface 13 is configured to receive a user's instruction from an input device connected to the client terminal 10 and to output information to an output device connected to the client terminal 10.

The input device is, for example, a keyboard, a pointing device, a touch panel, or a combination thereof.

The output device is, for example, a display.

The communication interface 14 is configured to control communication between the client terminal 10 and the server 30.

### (1-2) Server configuration

The configuration of the server 30 will be described with reference to FIG. 1.

As shown in FIG. 1, the server 30 includes a memory 31, a CPU 32, and a communication interface 34.

The memory 31 is configured to store a program and data.

The Memory 31 is, for example, a combination of ROM, RAM, and storage (for example, flash memory or hard disk).

The program is, for example, an OS program, an application program for executing information processing, or the like.

The data includes, for example, the following data:
- Database referenced in information processing; and
- Data obtained by executing an information processing (that is, an execution result of an information processing)

The CPU 32 is configured to realize the function of the server 30 by starting a program stored in the memory 31.

The input/output interface 33 is configured to accept a user instruction from an input apparatus connected to the server 30 and to output information to an output apparatus connected to the server 30.

The input device is, for example, a keyboard, a pointing device, a touch panel, or a combination thereof.

The output device is, for example, a display.

The communication interface 34 is configured to control communication between the server 30, the client terminal 10, and the external database 70.

### (1-3) Function of application

The function of the application of the present embodiment is described.

FIG. 2 is a diagram illustrating functions of an application according to the present embodiment.

The CPU 12 in FIG. 1 implements the function of the skin care application 120 by executing a program stored in the memory 11.

As shown in FIG. 2, the skin care application 120 transmits living environment information to the server 30.

The server 30 transmits advice information corresponding to the information transmitted from the skin care application 120 to the skin care application 120.

The skin care application 120 displays the advice information transmitted from the server 30 on the display.

### (2) Database

The database of the present embodiment is described.

The following databases are stored in the memory 31.

### (2-1) User information database

The user information database of the present embodiment is described.

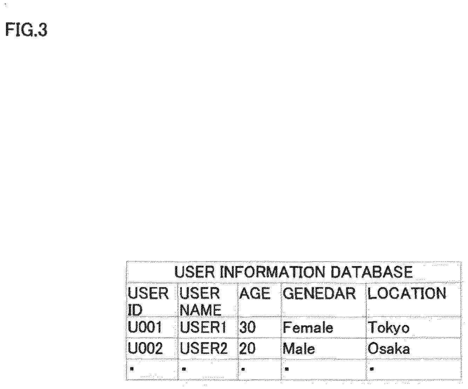

FIG. 3 is a diagram illustrating a data structure of the user information database according to the present embodiment.

The user information database stores information (hereinafter referred to as "user information") about users.

As shown in FIG. 3, the user information database includes a "USER ID" field, a "USER NAME" field, an "AGE" field, a "GENDER" field, and a "LOCATION" field.

Each field is associated with each other.

The "USER ID" field stores a user ID.

The user ID is information for identifying the user.

Information in the "USER ID" field is determined by the server 30.

In the "USER NAME" field, information (for example, text) related to the user name is stored.

Information in the "USER NAME" field is determined by the user.

The "AGE" field stores information indicating the age of the user.

Information in the "AGE" field is determined by the user.

In the "GENDER" field, information indicating the gender of the user is stored.

The information in the "GENDER" field is determined by the user.

The "LOCATION" field stores user position information.

The information in the "LOCATION" field is information determined by the user or information acquired from the client terminal 10 (for example, GPS (Global Positioning System) information).

### (2-2) Living environment information database

The living environment information database of the present embodiment is described.

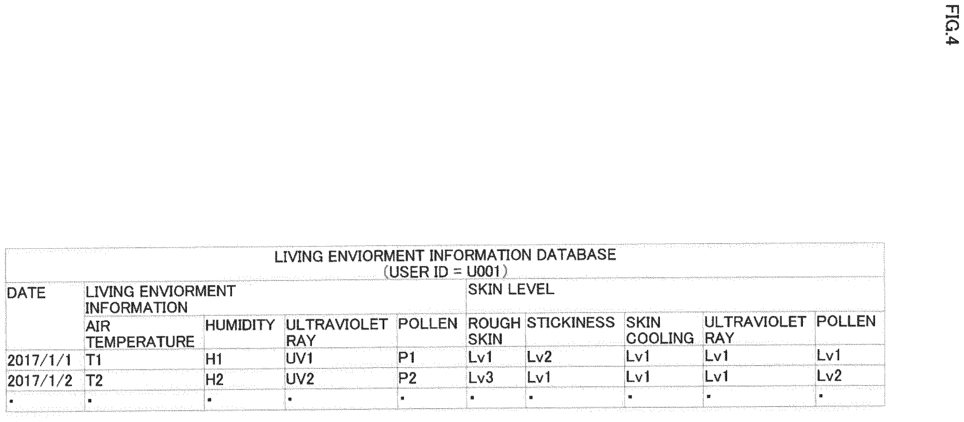

FIG. 4 is a diagram illustrating a data structure of the living environment information database according to the present embodiment.

Living environment information is stored in the living environment information database.

As shown in FIG. 4, the living environment information database is associated with a user ID.

The living environment information database includes a "DATE" field, a "LIVING ENVIRONMENT INFORMATION" field, and a "SKIN LEVEL" field.

Each field is associated with each other.

In the "DATE" field, information indicating a date corresponding to the living environment information is stored.

The "LIVING ENVIRONMENT INFORMATION" field includes a "air temperature" field, a "humidity" field, an "ultraviolet ray" field, and a "pollen" field.

The "air temperature" field stores information indicating the air temperature of the user's living environment.

The "humidity" field stores information indicating the humidity of the user's living environment.

The "ultraviolet ray" field stores information indicating the ultraviolet index of the user's living environment.

The "pollen" field stores information indicating the amount of scattered pollen by the user's living environment.

The "SKIN LEVEL" field includes a "rough skin" field, a "stickiness" field, a "skin cooling" field, an "ultraviolet ray" field, and a "pollen" field.

In the "rough skin" field, information indicating the determination result of the skin damage level caused by rough skin is stored.

The "stickiness" field stores information indicating the determination result of the skin damage level caused by the skin stickiness.

In the "skin cooling" field, information indicating the determination result of the skin damage level caused by the skin cooling is stored.

In the "ultraviolet ray" field, information indicating the determination result of the skin damage level caused by ultraviolet ray is stored.

The "pollen" field stores information indicating the determination result of the skin damage level caused by pollen.

It means that the higher the skin level of each field, the greater the damage of the skin.

FIG. 4 shows that on January 1, 2017, the air temperature is T1, the humidity is H1, the ultraviolet index is UV1, the pollen scattering amount is P1, the skin level related to rough skin is Lv1, and the skin level related to stickiness is Lv2, the skin level related to cool skin is Lv1, the skin level related to ultraviolet rays is Lv1, and the skin level related pollen is Lv1.

### (2-3) Advice information database

The advice information database of the present embodiment is described.

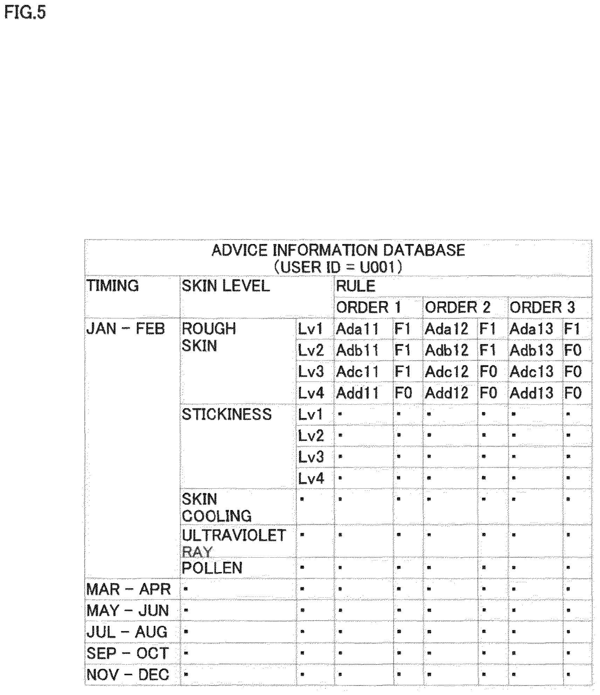

FIG. 5 is a diagram illustrating a data structure of the advice information database according to the present embodiment.

Advice information is stored in the advice information database.

As shown in FIG. 5, the advice information database is associated with the user ID.

The advice information database includes a "TIMING" field, a "SKIN LEVEL" field, and a "RULE" field.

Each field is associated with each other.

The "TIMING" field stores information related to the timing when advice information is provided.

The "SKIN LEVEL" field stores information indicating the skin level of each category of skin level ("rough skin", "stickiness", "skin cooling", "ultraviolet rays", and "pollen").

The "RULE" field includes an "order 1" field to an "order 3" field.

In the "order 1" field to the "order 3" field, an advice ID for identifying advice information and a flag are stored, respectively.

The advice information includes, for example, at least one of information related to an explanation of the skin condition and information related to a skin care proposal.

The flag is information indicating whether or not the advice information has been presented.

The flag "F0" indicates that advice information has not been presented.

The flag "F1" indicates that the advice information has been presented.

That is, the order of presentation is assigned to the advice information for each skin level.

The advice information to be presented next is specified by the flag.

FIG. 5 shows the following example.

For advice information corresponding to the skin level "Lv1" of "rough skin" in the timing "January to February", the advice information identified by the advice ID "Ada 1 1 ", the advice information identified by "Ada12", the advice information identified by the advice ID "Adal3" are presented in this order. - For advice information corresponding to the skin level "Lv2" of "rough skin" in the timing "January to February", the advice information identified by the advice IDs "Adb11" and "Adb12" has been presented, and the advice information identified by the ID "Adb13" has not been presented (the advice information to be presented next is the advice information identified by the advice ID "Adb13").

### (3) Flow of information processing

The flow of the information processing of the present embodiment is described.

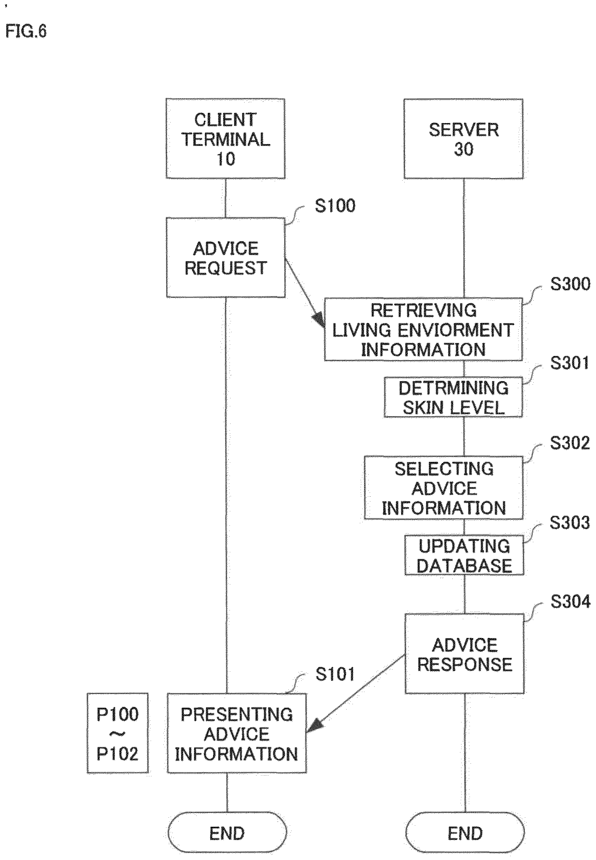

FIG. 6 is a flowchart of the information processing of the present embodiment.

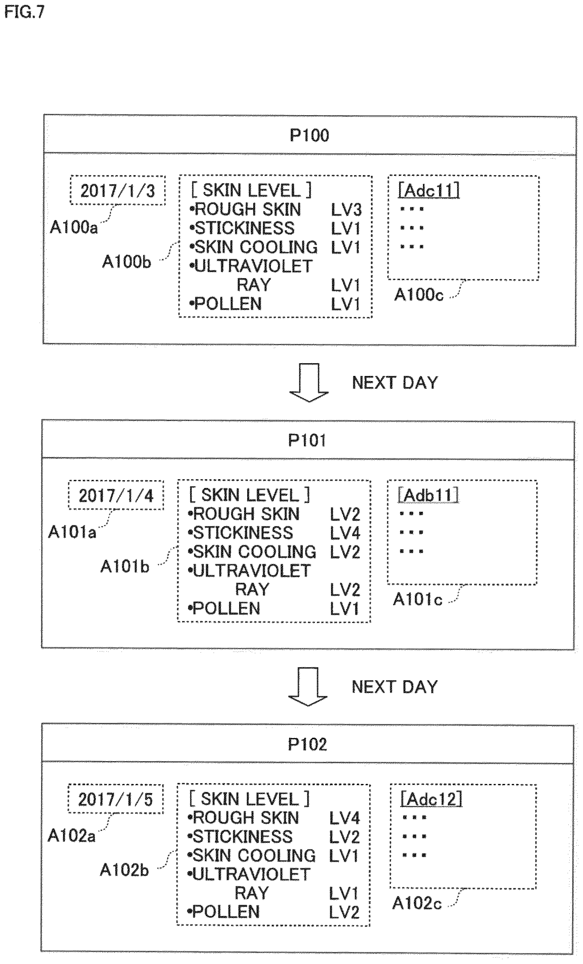

FIG. 7 is a diagram illustrating an example of a screen displayed in the information processing according to the present embodiment.

The information processing of the present embodiment is executed by the client terminal 10 and the server 30.

Each step of processing of the client terminal 10 is a function of the skin care application 120.

As shown in FIG. 6, the client terminal 10 executes an advice request (S100).

Specifically, when the user performs a predetermined operation on the client terminal 10 (for example, an instruction to activate the skin care application 120), the CPU 12 transmits advice request data to the server 30.

The advice request data includes a user ID.

After the step S100, the server 30 executes retrieving living environment information (S300).

Specifically, the CPU 32 specifies information in the "LOCATION" field associated with the user ID included in the advice request data in the user information database (FIG. 3).

Next, the CPU 32 retrieves, from the external database 70, living environment information (for example, information indicating air temperature, information indicating humidity, ultraviolet index, and information indicating the amount of scattered pollen) corresponding to the information in the specified "LOCATION" field).

After the step S300, the server 30 executes determining skin level (S301).

Specifically, the CPU 32 specifies information in the "AGE" field associated with the user ID included in the advice request data in the user information database (FIG. 3).

Next, CPU32 determines the skin level of rough skin based on the information which shows the humidity contained in advice request data, and the ultraviolet-ray index retrieved in the step S300.

Next, the CPU 32 determines the skin level of stickiness based on living environment information (that is, information indicating air temperature and information indicating humidity) included in the advice request data.

Next, CPU32 determines the skin level of skin cooling based on the information which shows the air temperature contained in advice request data.

Next, CPU32 determines the skin level of ultraviolet-ray based on the ultraviolet-ray index retrieved by the step S300.

Next, CPU32 determines the skin level of pollen based on the information which shows the amount of pollen scattering retrieved in the step S300.

After step S301, the server 30 executes selecting advice information (S302).

Specifically, the CPU 32 specifies the category having the highest skin level determined in the step S301 as the category for which advice information is to be presented.

As an example, when the skin level of "rough skin" is the highest, the CPU 32 specifies the category "rough skin".

Next, the CPU 32, in the advice information database (FIG. 5), in the order of the "order 1" field to the "order 3" field of the identified category, the advice ID corresponding to the flag "F0" (that is, advice information to be presented next) is specified as an advice ID for identifying advice information to be presented.

As an example, the CPU 32 specifies the advice ID "Adc12" in the "order 2" field for the advice information corresponding to the skin level "Lv3" of "rough skin" in the timing "January to February" in FIG. 5.

After the step S302, the server 30 executes updating database (S303).

Specifically, the CPU 32 stores information indicating the execution date of the information processing in the "DATE" field in the living environment information database (FIG. 4), and stores the living environment information (information indicating air temperature, humidity, ultraviolet index, and scattered pollen) referred to in the step S301 in the "LIVING ENVIRONMENT INFORMATION" field, and stores the skin level (the skin level of rough skin, the skin level of stickiness, the skin level of skin cooling, the skin level of ultraviolet-ray, and the skin level of pollen) determined in the step.

Next, the CPU 32 stores the flag "F1" in the "flag" field corresponding to the advice ID specified in the step S302 in the advice information database (FIG. 5).

After the step S303, the server 30 executes an advice response (S304).

Specifically, the CPU 32 transmits an advice response data to the client terminal 10.

The advice response data includes information indicating the skin level determined in the step S301 and advice information identified by the advice ID specified in the step S302.

After the step S304, the client terminal 10 executes presenting advice information (S101).

Specifically, the CPU 12 displays the screen P100 (FIG. 7) on the display.

As shown in FIG. 7, the screen P100 includes areas A100a to A100c.

In the area A100a, the execution date of information processing is displayed.

In the area A100b, information indicating the skin level included in the advice response data is displayed for each category.

In the area A100c, advice information included in the advice response data is displayed.

The screen P100 shows an example in which advice information (advice ID "Adc12") corresponding to the skin level of rough skin "LV3" is presented on January 3, 2017.

A screen P101 shows an example in which advice information (advice ID "Adbll") corresponding to the skin level of stickiness "LV4" is presented on January 4, 2017.

The screen P102 shows an example in which advice information (advice ID "Adc12") corresponding to the skin level of rough skin "LV4" is presented on January 5, 2017.

### (4) Variations

Variations of the present embodiment are described.

### (4-1) Variation 1

Variation 1 of the present embodiment is described.

Variation 1 is an example in which statistical information on the skin level of each user is presented.

The CPU 32 of the first variation generates statistical information on the information in the "SKIN LEVEL" field of the living environment information database (FIG. 4) for each user information (for example, "AGE" field, "sex" field, and "LOCATION" field information) of the user information database (FIG. 3).

For example, the statistical information includes at least one of information indicating a ranking of the skin level of each user, an average value of the skin level of each user, and a maximum value and a minimum value of the skin level of each user.

Next, the CPU 32 transmits the generated statistical information to the client terminal 10.

Next, the CPU 12 displays a screen including statistical information transmitted from the server 30 on the display.

According to the variation 1, the user can easily compare the user's skin level with the skin level of another user.

### (4-2) Variation 2

A variation 2 of the present embodiment is described.

Variation 2 is an example in which living environment information is retrieved from an apparatus other than the external database 70.

The communication interface 14 of Variation 2 is connected to a wearable apparatus.

The wearable apparatus is configured to acquire the living environment information of the user.

As an example, when the wearable apparatus acquires all the living environment information, the CPU 12 of Variation 2 retrieves the living environment information from the wearable apparatus before the step S100 in FIG 6.

In this case, the advice request data includes living environment information in addition to the information retrieved in the step S100.

The CPU 32 omits the step S300 and executes the step S301.

As another example, when the wearable apparatus acquires some living environment information (for example, information indicating the air temperature and information indicating the humidity), the CPU 12 of the variation 2 retrieves at least part of living environment information from wearable devices before the step S100 in FIG. 6.

In this case, the advice request data includes at least part of living environment information in addition to the information retrieved in the step S100.

In the step S300, the CPU 32 retrieves the remaining living environment information (for example, information indicating the ultraviolet index and the amount of scattered pollen).

According to the variation 2, it is possible to provide advice information more suitable for the user's living environment.

### (4-3) Variation 3

A variation 3 of the present embodiment is described.

Variation 3 is an example in which a plurality of pieces of advice information is presented.

In the selecting advice information (S302) in FIG. 6, the CPU 32 of the Variation 3 identifies a category whose skin level determined in the step S301 is equal to or greater than a predetermined threshold as a category for which advice information is to be presented.

This threshold indicates the skin level for determining whether or not the advice information is necessary.

Next, the CPU 32, in the advice information database (FIG. 5), in the order of the "order 1" field to the "order 3" field of the identified category, specifies the advice ID corresponding to the flag "F0" (that is, to be presented next) as the advice ID identifying advice information to be presented.

According to the third variation, it is possible to provide advice information regarding a category having a certain damage level or higher.

### (5) Summary of the present embodiment

This embodiment is summarized.

The information processing apparatus (30) of the present embodiment includes:
a retrieve module (S300) configured to retrieve living environment information related to the user's living environment;
a determination module (S301) configured to determine a skin level based on the retrieved living environment information;
a selection module (S302) configured to select advice information associated with the determined skin level from a plurality of pieces of advice information associated with the skin level, the advice information including advice related to the user's skin care; and
a presentation module (S304) configured to present the selected advice information.

Thereby, the advice according to a user's living environment can be provided to a user.

The selection module (S302) may select the advice information so that the same advice information is not continuously presented.

The selection module (S302) may select advice information according to a rule indicating the order assigned to each of the plurality of advice information.

Thereby, the content of advice can be changed every time advice is provided.

The selection module (S302) may further select advice information associated with the timing when the advice information is presented from a plurality of pieces of advice information associated with the timing.

Thereby, the advice according to the timing when the advice is presented can be provided.

The selection module (S302) may select advice information according to a rule indicating an order assigned to each of a plurality of pieces of advice information associated with one timing.

Thereby, the variation of the advice provided to the user can be expanded.

The selection module may further select advice information associated with the age of the user among a plurality of pieces of advice information associated with the age.

Thereby, the advice according to a user's age can be provided.

The living environment information may include at least one of information on air temperature, information on humidity, information indicating the intensity of ultraviolet rays, and information on the amount of scattered pollen.

The determination module (S301) may determine the level of rough skin based on a combination of the intensity of ultraviolet ray and humidity.

The determination module (S301) may determine the level of skin stickiness based on a combination of air temperature and humidity.

### (6) Other Variations

The memory 11 may be connected to the client terminal 10 via the network NW.

The memory 31 may be connected to the server 30 via the network NW.

Each step of the information processing may be executed by either the client terminal 10 or the server 30.

The client terminal 10 may execute all the steps, or the server 30 may execute all the steps.

### [Reference Signs List]

1: Order
1: Information processing system
3: Order
10: Client terminal
11: Memory
12: CPU
13: Input/output interface
14: Communication interface
30: Server
31: Memory
32: CPU
33: Input/output interface
34: Communication interface
70: External database
120: Skin care application

## Claims

1. A computer system comprising: a client terminal which a user uses and a server,
the server includes:
a storage configured to store living environment information database associated with user ID including living environment information, and advice information database associated with the user ID including advice information and rule of presentation to the advice information;
a retrieve module configured to retrieve living environment information related to the user's living environment, the living environment information stored in the storage and associated with the user's ID sent from the client terminal;
a determination module configured to determine the user's skin level based on the retrieved living environment information;
a selection module configured to select advice information associated with the determined user's skin level among a plurality of pieces of advice information associated with the skin level according to the rule so that the same advice information is not continuously presented, each piece of advice information including advice related to the user's skin care; and
a presentation module configured to present the selected advice information on a display of the client terminal;
wherein the selection module selects the advice information according to the rule indicating an order assigned to each of the plurality of pieces of advice information.

2. The computer system according to any one of claim 1, wherein the selection module further selects advice information associated with a timing when the advice information is presented among a plurality of pieces of advice information associated with the timing.

3. The computer system according to claim 2, wherein the selection module selects the advice information according to a rule indicating an order assigned to each of a plurality of pieces of advice information associated with one timing.

4. The computer system according to any one of claims 1 to 3, wherein the selection module further selects advice information associated with the age of the user among a plurality of pieces of advice information associated with the age.

5. The computer system according to any one of claims 1 to 4, wherein the living environment information includes at least one of information on air temperature, information on humidity, information indicating the intensity of ultraviolet rays, and information on the amount of scattered pollen.

6. The computer system according to claim 5, wherein the determination module determines a skin level of rough skin based on a combination of the intensity of the ultraviolet rays and the humidity.

7. The computer system according to claim 5 or 6, wherein the determination module determines a level of skin stickiness based on a combination of the air temperature and the humidity.

8. A program for causing a computer to function as each modules according to any one of claims 1 to 7.

## Patentansprüche

1. Computersystem, umfassend: ein Client-Endgerät, das ein Benutzer verwendet, und einen Server,
wobei der Server enthält:
einen Speicher, der konfiguriert ist, eine mit einer Benutzer-ID verknüpfte Lebensumfeldinformationen-Datenbank, die Lebensumfeldinformationen enthält, und eine mit der Benutzer-ID verknüpfte Ratschlaginformationen-Datenbank, die Ratschlaginformationen und eine Regel der Darstellung der Ratschlaginformationen enthält, zu speichern;
ein Abrufmodul, das konfiguriert ist, Lebensumfeldinformationen abzurufen, die sich auf das Lebensumfeld des Benutzers beziehen, wobei die Lebensumfeldinformationen in dem Speicher gespeichert und mit der Benutzer-ID verknüpft sind, die von dem Client-Gerät gesendet wird;
ein Bestimmungsmodul, das konfiguriert ist, das Hautniveau bzw. den Hautzustand des Benutzers basierend auf den abgerufenen Lebensumfeldinformationen zu bestimmen;
ein Auswahlmodul, das konfiguriert ist, Ratschlaginformationen, die mit dem bestimmten Hautzustand des Benutzers verknüpft sind, aus einer Mehrzahl von Ratschlaginformationen auszuwählen, die mit dem Hautzustand verknüpft sind, und zwar gemäß der Regel, so dass nicht kontinuierlich dieselben Ratschlaginformationen dargestellt werden, wobei jede Ratschlaginformation Ratschläge zur Hautpflege des Benutzers enthält; und ein Darstellungsmodul, das konfiguriert ist, die ausgewählten Ratschlaginformationen auf einem Display des Client-Geräts darzustellen;
wobei das Auswahlmodul die Ratschlaginformationen gemäß der Regel auswählt, die eine Reihenfolge angibt, die jeder der Mehrzahl von Ratschlaginformationen zugewiesen ist.

2. Computersystem nach einem der Ansprüche 1, wobei das Auswahlmodul ferner Ratschlaginformationen, die mit einem Zeitpunkt verknüpft sind, zu dem die Ratschlaginformationen dargestellt werden, aus einer Mehrzahl von Ratschlaginformationen auswählt, die mit dem Zeitpunkt verknüpft sind.

3. Computersystem nach Anspruch 2, wobei das Auswahlmodul die Ratschlaginformationen gemäß einer Regel auswählt, die eine Reihenfolge angibt, die jeder einer Mehrzahl von Ratschlaginformationen zugewiesen ist, die mit einem Zeitpunkt verknüpft sind.

4. Computersystem nach einem der Ansprüche 1 bis 3, wobei das Auswahlmodul ferner Ratschlaginformationen, die mit dem Alter des Benutzers verknüpft sind, aus einer Mehrzahl von Ratschlaginformationen auswählt, die mit dem Alter verknüpft sind.

5. Computersystem nach einem der Ansprüche 1 bis 4, wobei die Lebensumfeldinformationen zumindest eines von Lufttemperaturinformationen, Luftfeuchtigkeitsinformationen, Informationen zur Intensität der ultravioletten Strahlen und Informationen zur Menge der verteilten Pollen enthalten.

6. Computersystem nach Anspruch 5, wobei das Bestimmungsmodul ein Hautrauheitsniveau bzw. einen Hautrauheitsgrad basierend auf einer Kombination aus der Intensität der ultravioletten Strahlen und der Luftfeuchtigkeit bestimmt.

7. Computersystem nach Anspruch 5 oder 6, wobei das Bestimmungsmodul ein Hautklebrigkeitsniveau bzw. einen Hautklebrigkeitsgrad basierend auf einer Kombination aus der Lufttemperatur und der Luftfeuchtigkeit bestimmt.

8. Programm zum Veranlassen eines Computers, als jedes Modul nach einem der Ansprüche 1 bis 7 zu fungieren.

## Revendications

1. Système informatique comprenant : un terminal client qu'utilise un utilisateur et un serveur, le serveur inclut :
un stockage configuré pour stocker une base de données d'informations de milieu de vie associée à un identifiant utilisateur incluant des informations de milieu de vie, et une base de données d'informations de conseil associée à l'identifiant utilisateur incluant des informations de conseil et une règle de présentation concernant les informations de conseil ;
un module de récupération configuré pour récupérer des informations de milieu de vie liées au milieu de vie de l'utilisateur, les informations de milieu de vie étant stockées dans le stockage et associées à l'identifiant de l'utilisateur envoyé du terminal client ;
un module de détermination configuré pour déterminer le niveau cutané de l'utilisateur en fonction des informations de milieu de vie récupérées ;
un module de sélection configuré pour sélectionner des informations de conseil associées au niveau cutané déterminé de l'utilisateur parmi une pluralité d'informations de conseil associées au niveau cutané en fonction de la règle de sorte que les mêmes informations de conseil ne sont pas présentées en continu, chaque information de conseil incluant des conseils liés aux soins de la peau de l'utilisateur ; et
un module de présentation configuré pour présenter les informations de conseil sélectionnées sur un affichage du terminal client ;
dans lequel le module de sélection sélectionne les informations de conseil en fonction de la règle indiquant un ordre attribué à chacune de la pluralité d'informations de conseil.

2. Système informatique selon l'une quelconque de la revendication 1, dans lequel le module de sélection sélectionne en outre des informations de conseil associées à un moment auquel les informations de conseil sont présentées parmi une pluralité d'informations de conseil associées au moment.

3. Système informatique selon la revendication 2, dans lequel le module de sélection sélectionne les informations de conseil en fonction d'une règle indiquant un ordre attribué à chacune d'une pluralité d'informations de conseil associées à un moment.

4. Système informatique selon l'une quelconque des revendications 1 à 3, dans lequel le module de sélection sélectionne en outre des informations de conseil associées à l'âge de l'utilisateur parmi une pluralité d'informations de conseil associées à l'âge.

5. Système informatique selon l'une quelconque des revendications 1 à 4, dans lequel les informations de milieu de vie incluent au moins une d'informations sur la température de l'air, d'informations sur l'humidité, d'informations indiquant l'intensité de rayons ultraviolets et d'informations sur la quantité de pollen dispersé.

6. Système informatique selon la revendication 5, dans lequel le module de détermination détermine un niveau cutané de peau rêche en fonction d'une combinaison de l'intensité des rayons ultraviolets et de l'humidité.

7. Système informatique selon la revendication 5 ou 6, dans lequel le module de détermination détermine un niveau de moiteur cutanée en fonction d'une combinaison de la température de l'air et de l'humidité.

8. Programme pour amener un ordinateur à fonctionner en tant que chaque module selon l'une quelconque des revendications 1 à 7.
